# EUROPEAN PATENT APPLICATION

(11) **EP 4 400 589 A1**
(43) Date of publication of application: **17.07.2024**
(21) Application number: 22867379.4
(22) Date of filing: 07.09.2022
(51) Int. Cl.: C12N 15/56, A23C 7/04, A23C 9/12, A23C 21/02, A23C 23/00, A23L 33/00, C12N 1/15, C12N 1/19, C12N 1/21, C12N 5/10, C12N 9/38, C12N 15/63

(54) **LACTASE, LACTASE PREPARATION, GENE, RECOMBINANT VECTOR, AND TRANSFORMANT**

(30) Priority: 07.09.2021 JP 2021145630; 04.03.2022 JP 2022033806
(71) Applicant: Godo Shusei Co., Ltd., Matsudo-shi, Chiba 271-0064 (JP)
(72) Inventor: ODAKA, Rei, Matsudo-shi, Chiba 271-0064 (JP); SANO, Ryoko, Matsudo-shi, Chiba 271-0064 (JP); IWASAKI, Takumi, Matsudo-shi, Chiba 271-0064 (JP); BABA, Masahiro, Matsudo-shi, Chiba 271-0064 (JP); SABURI, Wataru, Sapporo-shi, Hokkaido 060-0809 (JP); MORI, Haruhide, Sapporo-shi, Hokkaido 060-0809 (JP)
(74) Representative: Hoffmann Eitle
(86) International application number: PCT/JP2022/033563
(87) International publication number: WO 2023/038057

(57) **Abstract**

Provided is a lactase preparation having an excellent lactose decomposing action in milk. A lactase according to an aspect of the present invention has, as lactase activity, any one of the following activities (1) to (6) or a combination of two or more thereof:
(1) when the lactase is added to cow's milk, an added amount of protein in the lactase at which a residual lactose concentration reaches 0.1% or less after 16 hours at 10°C is 15.1 mg/L or less;
(2) when the lactase is added to cow's milk, the added amount of protein in the lactase at which the residual lactose concentration reaches 0.01% or less after 16 hours at 10°C is 30.2 mg/L or less;
(3) when the lactase is added to cow's milk, the added amount of protein in the lactase at which the residual lactose concentration reaches 0.1% or less after 24 hours at 10°C is 10.1 mg/L or less;
(4) when the lactase is added to cow's milk, the added amount of protein in the lactase at which the residual lactose concentration reaches 0.01% or less after 24 hours at 10°C is 15.1 mg/L or less;
(5) when the lactase is added to cow's milk in an amount that makes a final concentration 1.4 LYU/ml, the residual lactose concentration reaches 0.1% or less after 24 hours at 10°C; and
(6) when the lactase is added to cow's milk in the amount that makes a final concentration 2.1 LYU/ml, the residual lactose concentration reaches 0.01% or less after 24 hours at 10°C.

## Description

### Technical Field

The present invention relates to a lactase, a lactase preparation, a gene, a recombinant vector, and a transformant.

### Background Art

From the past, cow's milk has been used for a long time as a nutritious and useful food. Cow's milk contains lactose, a type of sugar. Lactose is decomposed in the intestine by lactase, but in some humans, the amount of lactase secreted into the intestine decreases as they grow, and therefore when a large amount of cow's milk or a cow's milk processing product (hereinafter, collectively referred to as "dairy products") is ingested, so-called lactose intolerance such as abdominal pain or diarrhea is caused. This has been a cause of hindering a wide intake of this nutritious food.

In recent years, dairy products with previously reduced lactose have been provided. Such dairy products can be ingested without problems even by a human with lactose intolerance.

Reduction of lactose has been carried out in various ways. For example, there is a method in which milk before production of cow's milk is treated with a lactase preparation to hydrolyze lactose in cow's milk (see, for example, Patent Literature 1).

### Citation List

### Patent Literature

Patent Literature 1: JP 2004-534527 A

### Summary of Invention

### Technical Problem

The conventional lactase preparation has a problem that lactose decomposing action stops as the lactose decomposition in milk progresses. Therefore, in order to achieve lactose-free milk (lactose concentration: 0.1 mass% or 0.01 mass%), it is necessary to enhance the lactose decomposing action.

The present invention has been made in view of the problem described above, and an object of the present invention is to provide a lactase and a lactase preparation which are excellent in lactose decomposing action in milk (particularly in cow's milk).

### Solution to Problem

One aspect of the present invention is lactase. The lactase has, as lactase activity, any one of the following activities (1) to (6) or a combination of two or more thereof:
(1) when the lactase is added to cow's milk, an added amount of protein in the lactase at which a residual lactose concentration reaches 0.1% or less after 16 hours at 10°C is 15.1 mg/L or less;
(2) when the lactase is added to cow's milk, the added amount of protein in the lactase at which the residual lactose concentration reaches 0.01% or less after 16 hours at 10°C is 30.2 mg/L or less;
(3) when the lactase is added to cow's milk, the added amount of protein in the lactase at which the residual lactose concentration reaches 0.1% or less after 24 hours at 10°C is 10.1 mg/L or less;
(4) when the lactase is added to cow's milk, the added amount of protein in the lactase at which the residual lactose concentration reaches 0.01% or less after 24 hours at 10°C is 15.1 mg/L or less;
(5) when the lactase is added to cow's milk in an amount that makes a final concentration 1.4 LYU/ml, the residual lactose concentration reaches 0.1% or less after 24 hours at 10°C; and
(6) when the lactase is added to cow's milk in the amount that makes a final concentration 2.1 LYU/ml, the residual lactose concentration reaches 0.01% or less after 24 hours at 10°C.

In the lactase of the above aspect, the lactase may be selected from any one of the following (i) to (iii):
(i) a protein consisting of an amino acid sequence represented by SEQ ID NO:1;
(ii) a protein consisting of an amino acid sequence in which one to several amino acid residues of the amino acid sequence represented by SEQ ID NO:1 are deleted, substituted, or inserted, and having lactase activity; and
(iii) a protein consisting of an amino acid sequence having 70% or more of identity to the amino acid sequence represented by SEQ ID NO: 1 and having lactase activity.

In the lactase of the above aspect, the cow's milk may satisfy one or more of the following requirements:
(A) a solid content contained in cow's milk is 0.1 mass% or more and 30 mass% or less;
(B) an amount of lactose contained in cow's milk is 0.1 mass% or more and 30 mass% or less;
(C) an amount of protein contained in cow's milk is 0.1 mass% or more and 30 mass% or less; and
(D) an amount of fat contained in cow's milk is 0.1 mass% or more and 30 mass% or less.

Another aspect of the present invention is a lactase preparation. The lactase preparation includes the lactase described above, and
at least one selected from water, a salt, an excipient, a suspension agent, a buffer agent, a stabilizer, a preservative, or a physiological saline. The salt may be one or more selected from a group consisting of magnesium chloride, sodium chloride, potassium chloride, calcium chloride, and manganese chloride. The lactase preparation may contain the lactase described above and a second lactase having a different origin or different properties from the lactase.

Still another aspect of the present invention is a gene encoding the following protein (i), (ii), or (iii):
(i) a protein consisting of an amino acid sequence represented by SEQ ID NO: 1;
(ii) a protein consisting of an amino acid sequence in which one to several amino acid residues of the amino acid sequence represented by SEQ ID NO: 1 are deleted, substituted, or inserted, and having lactase activity; and
(iii) a protein consisting of an amino acid sequence having 70% or more of identity to the amino acid sequence represented by SEQ ID NO: 1 and having lactase activity.

The gene of the above aspect may include any one of the following DNAs (a) to (d):
(a) a DNA consisting of a base sequence set forth in SEQ ID NO:2;
(b) a DNA consisting of a base sequence in which one to several bases are deleted, substituted, or added in the base sequence set forth in SEQ ID NO:2 and encoding the protein having lactase activity;
(c) a DNA consisting of a base sequence having 70% or more of sequence identity to the base sequence set forth in SEQ ID NO:2 and encoding the protein having lactase activity; and
(d) a DNA that hybridizes with a DNA consisting of a base sequence complementary to the base sequence set forth in SEQ ID NO:2 under stringent conditions and encodes the protein having lactase activity.

SEQ ID NO:2 represents a base sequence including a stop codon.

Still another aspect of the present invention is a recombinant vector having the gene described above.

Still another aspect of the present invention is a transformant transformed with the recombinant vector described above.

Still another aspect of the present invention is a method for producing a lactose-decomposed milk. The method for producing a lactose-decomposed milk includes a step of adding the lactase or the lactase preparation described above to a raw material milk; and
a step of reacting the lactase or a lactase contained in the lactase preparation with the raw material milk for a predetermined time.

### Advantageous Effects of Invention

According to the present invention, it is possible to provide a technique relating to a lactase and a lactase preparation which are excellent in lactose decomposing action in milk (particularly in cow's milk).

### Brief Description of Drawings

Fig. 1 is a graph showing changes in residual lactose concentrations at 10°C when BL105A_1453 and other lactase preparations are added to 1 mL of cow's milk in an amount such that the amount of protein is about 15.1 µg.
Fig. 2 is a graph enlarging a range of a horizontal axis (10 to 50 hours) and a vertical axis (lactose concentration in cow's milk: 0 to 0.20%) in Fig. 1.
Fig. 3 is graphs showing lactose decomposition rates (reaction rates) when various salts having different concentrations are added to a BL105A_1453 purified enzyme liquid.
Fig. 4 is a graph showing an optimum pH of a BL105A_1453 purified enzyme liquid.
Fig. 5 is a graph showing a pH stability of a BL105A_1453 purified enzyme liquid.
Fig. 6 is a graph showing an optimum temperature of a BL105A_1453 purified enzyme liquid.
Fig. 7 is a graph showing a temperature stability of a BL105A_1453 purified enzyme liquid.
Fig. 8 is graphs showing analysis of main structures of products by HPAEC-PAD.
Fig. 9 is a graph showing changes in lactose concentrations during fermentation stage and storage when each lactase and a starter are simultaneously added to milk.
Fig. 10 is a graph showing changes in the lactose concentrations when YNL and BL105A_1453 are added separately or in combination to milk.

### Description of Embodiments

Hereinafter, embodiments of the present invention will be described in detail. In the present description, a notation "a to b" in the description of the numerical range represents "a or more and b or less" unless otherwise specified.

### <Lactase>

The lactase according to the embodiments has, as lactase activity, any one of the following activities (1) to (6) or a combination thereof: preferably two or more thereof, more preferably three or more thereof, still more preferably four or more thereof, and particularly preferably five or more thereof. Most preferably, all of (1) to (6) are satisfied:
(1) when the lactase is added to cow's milk, an added amount of protein in the lactase at which a residual lactose concentration reaches 0.1% after 16 hours at 10°C is 15.1 mg/L or less;
(2) when the lactase is added to cow's milk, the added amount of protein in the lactase at which the residual lactose concentration reaches 0.01% after 16 hours at 10°C is 30.2 mg/L or less;
(3) when the lactase is added to cow's milk, the added amount of protein in the lactase at which the residual lactose concentration reaches 0.1% after 24 hours at 10°C is 10.1 mg/L or less;
(4) when the lactase is added to cow's milk, the added amount of protein in the lactase at which the residual lactose concentration reaches 0.01% after 24 hours at 10°C is 15.1 mg/L or less;
(5) when the lactase is added to cow's milk in an amount that makes a final concentration 1.4 LYU/ml, the residual lactose concentration reaches 0.1% or less after 24 hours at 10°C; and
(6) when the lactase is added to cow's milk in the amount that makes a final concentration 2.1 LYU/ml, the residual lactose concentration reaches 0.01% or less after 24 hours at 10°C.

The lactase amounts in (1) to (4) are values calculated by Bradford method.

The lactase according to the embodiments is selected from any one of the following (i) to (iii):
(i) a protein consisting of an amino acid sequence represented by SEQ ID NO: 1;
(ii) a protein consisting of an amino acid sequence in which one to several amino acid residues of the amino acid sequence represented by SEQ ID NO: 1 are deleted, substituted, or inserted, and having lactase activity; and
(iii) a protein consisting of an amino acid sequence having 70% or more of identity to the amino acid sequence represented by SEQ ID NO: 1 and having lactase activity.

The (i) is a protein having lactase activity as to be described later.

The method for measuring the lactase activity is not particularly limited, and a known method can be freely selected. For example, in the present embodiment in which the lactase activity can be measured by the method to be described later, having lactase activity means that when the amount of protein contained in a measurement target is 1 mg, the lactase activity in the method to be described later is 1 LYU/mg-protein or more.

The protein consisting of the amino acid sequence represented by SEQ ID NO: 1 (hereinafter, may be referred to as BL105A_1453 or 1453) is a lactase derived from Bifidobacterium longum 105-A strain. The lactase is mentioned as a preferable example because it has an extremely high lactose decomposing action.

The number of amino acid residue deletions, substitutions, or insertions (hereinafter, may be referred to as the number of deletions and the like) in an amino acid sequence having one to several amino acid residues deleted, substituted, or inserted in the amino acid sequence represented by SEQ ID NO:1 is preferably 1 to 20, more preferably 1 to 10, still more preferably 1 to 8. The number of amino acid residue deletions and the like in the amino acid sequence represented by SEQ ID NO: 1 preferably indicates an enzyme activity equivalent to that of lactase consisting of the amino acid sequence represented by SEQ ID NO: 1.

In the present embodiment, the sequence identity with the amino acid sequence represented by SEQ ID NO:1 is preferably 70% or more, more preferably 80% or more, still more preferably 85% or more, particularly preferably 90% or more, still particularly preferably 95% or more, and most preferably 99% or more. Percentage identity of such sequences can be calculated using publicly or commercially available software with algorithms that compare a reference sequence as a query sequence. For example, BLAST, FASTA, GENETYX (manufactured by GENETYX CORPORATION), or the like can be used.

In the lactase according to the present embodiment, it is preferable to use a protein which is composed of an amino acid sequence having any of the above sequence identity and has lactase activity.

### <pH activity>

The protein consisting of the amino acid sequence represented by SEQ ID NO: 1 exhibits lactase activity in a pH range of 5.0 to 8.5, and has an optimum pH range of 5.5 to 6.5. The optimum pH is 6.0.

### <pH stability>

The protein consisting of the amino acid sequence represented by SEQ ID NO: 1 is a lactase that is stable at a pH in the range of 5.0 to 9.0. A pH of 5.2 to 8.4 is more stable and preferable.

### <Optimum temperature>

The protein consisting of the amino acid sequence represented by SEQ ID NO: 1 has lactase activity in a range of higher than 0°C to 60°C and higher lactase activity in a range of 20°C to 60°C, and has an optimum temperature range of 40°C to 55°C and an optimum temperature of 50°C.

### <Temperature stability>

The protein consisting of the amino acid sequence represented by SEQ ID NO: 1 is a lactase that exhibits thermal stability up to 50°C. Even after the protein is held at 50°C for 1 hour, the lactase residual activity is 100%.

### (Gene encoding lactase)

The gene according to the embodiment is a gene encoding the above-described protein having lactase activity, preferably a gene encoding the amino acid sequence of any one of the above (i) to (iii), and more preferably a gene consisting of DNA of any one of the following (a) to (d):
(a) a DNA consisting of a base sequence set forth in SEQ ID NO:2;
(b) a DNA consisting of a base sequence in which one to several bases are deleted, substituted, or added in the base sequence set forth in SEQ ID NO:2 and encoding a protein having lactase activity;
(c) a DNA consisting of a base sequence having 70% or more of sequence identity to the base sequence set forth in SEQ ID NO:2 and encoding a protein having lactase activity; and
(d) a DNA that hybridizes with a DNA consisting of a base sequence complementary to the base sequence set forth in SEQ ID NO:2 under stringent conditions and encodes a protein having lactase activity.

One to several bases in the base sequence in which one to several bases are deleted, substituted, or added in the base sequence set forth in SEQ ID NO:2 are preferably 1 to 10, more preferably 1 to 5, still more preferably 1 to 3, and further more preferably 1 or 2. In addition, deletion of a base means loss or disappearance of a base, substitution of a base means that a base is replaced with another base, and addition of a base means that a base is added. "Addition" includes addition of a base to one end or both ends of a sequence and insertion of another base between bases in the sequence.

In the gene of the present embodiment, the sequence identity of the base sequence is preferably 70% or more, more preferably 80% or more, still more preferably 85% or more, particularly preferably 90% or more, further particularly preferably 95% or more, and most preferably 99% or more.

The sequence identity of the base sequence can be determined using the algorithm BLAST (Pro. Natl. Acad. Sci. USA, 1993, 90: 5873-5877) by Karlin and Altschul. Based on this algorithm BLAST, programs called BLASTN and BLASTX have been developed (J. Mol. Biol., 1990, 215, p. 403-410). In addition, a homology analysis (Search homology) program of genetic information processing software Genetyx may be used. Specific methods of these analysis methods are known (see www.ncbi.nlm.nih.gov).

The stringent conditions refer to conditions under which base sequences having high identity are hybridized and base sequences having lower identity are not hybridized. The "stringent conditions" can be changed as appropriate depending on a level of identity required. The higher the stringent conditions are, the more highly identical sequences will hybridize. Examples of the stringent conditions include conditions described in Molecular Cloning: A Laboratory Manual (Second Edition, J. Sambrook et. al, 1989). In other words, examples thereof include conditions in which a solution containing 6 × SSC (Composition of 1 × SSC: 0.15 M sodium chloride solution, 0.015 M sodium citrate, pH 7.0), 0.5% SDS, 5 × Denhardt and 100 mg/mL herring sperm DNA is incubated at 65°C for 8 to 16 hours together with a probe and hybridized.

The protein (lactase) having lactase activity of the present embodiment can be obtained by a transformant obtained by introducing a vector containing the above-described gene encoding lactase into a microorganism according to a known method. The transformant may be retained in a form of a vector, or the gene may be retained in the genome. In other words, when the transformant is cultured in an appropriate medium, the vector contained in the transformant or the gene encoded on the genome is expressed, and the protein (lactase) having lactase activity of the present embodiment is produced. The lactase can be obtained by isolating and purifying the produced lactase from the culture.

The gene encoding the lactase of the present embodiment can be isolated from Bifidobacterium longum 105-A strain using any method that is used in the art. For example, the gene can be obtained by extracting the whole genome DNA of Bifidobacterium longum 105-A strain, then selectively amplifying a target gene by PCR using primers designed based on upstream and downstream gene sequences of a base sequence of SEQ ID NO:2, and purifying the amplified gene.

A type of vectors containing the gene encoding lactase of the present embodiment is not particularly limited, and examples thereof include vectors usually used for protein production, such as plasmids, cosmids, phages, viruses, YACs, and BACs. Among them, a plasmid vector is preferable, and a commercially available plasmid vector for protein expression, for example, pET, pBIC, or the like can be suitably used. Procedures for introducing genes into plasmid vectors are well known in the art.

In addition, examples of the microorganism for introducing a constructed vector described above include bacteria belonging to the genus Streptomyces, Brevibacillus, Staphylococcus, Enterococcus, Listeria, Bacillus, Escherichia, Saccharomyces, Shizosaccharomyces, Kluyveromyces, Aspergillus, Penicillium, Trichoderma, and eukaryotic microorganisms. As a method of introduction, a method commonly used in the art can be used.

When the obtained transformant is cultured in an appropriate medium, the gene introduced into the transformant is expressed, and the lactase of the present embodiment is produced. The culture medium to be used for culture can be appropriately selected by those skilled in the art according to types of transformant. The produced lactase of target can be isolated from the culture by an ordinary method and purified using a known purification method.

A lactase derived from Bifidobacterium longum 105-A strain has a high lactose decomposing action in cow's milk as shown in the examples to be described later.

### <Lactase preparation>

In addition to the active ingredient (lactase), the lactase preparation (lactase composition) of the present embodiment may contain, for example, water, a salt, an excipient, a suspension agent, a buffer agent, a stabilizer, a preservative, or a physiological saline.

Only one kind of lactase may be used, or a plurality of kinds thereof may be mixed and used. When lactases having different lactose decomposing actions are mixed and used for a subject containing lactose, it is easy to enjoy advantages of each lactase. For example, there may be a case where a plurality of lactases having different origins are combined, a case where lactase having a lactose decomposing action in a neutral region and lactase having a lactose decomposing action in an acidic region are used in combination, a case where lactase having a high initial lactose decomposition rate with a final lactose decomposition rate not reaching 99.9% and lactase having a low initial lactose decomposition rate with a final lactose decomposition rate of 99.9% or more are used in combination, and the like.

A first lactase and a second lactase having a different origin or different properties from the first lactase can be combined according to desired properties and used as a lactase preparation. The number of lactases to be mixed, such as a third lactase and a fourth lactase, is not limited. In a case where BL105A 1453 is used as the first lactase, the origin of this lactase is Bifidobacterium longum 105-A, and the final lactose decomposition rate reaches 99.9% or more. For this enzyme, a lactase of different origin or a lactase having a high initial decomposition rate in milk or the like can be used as the second lactase. For example, a yeast-derived lactase, a mold-derived lactase, or a lactic acid bacterium-derived lactase can be used. More specific examples thereof include lactases derived from Kluyveromyces lactis and Kluyveromyces marxianus, lactases derived from Aspergillus oryzae, and lactases derived from Lactobacillus delbrueckii.

In a case where a plurality of lactases are contained in the lactase preparation, a mass of the lactase protein may be used as a reference. In a case where the first lactase and the second lactase are contained, a mass ratio may be in a range of 1 : 99 to 99 : 1, preferably in a range of 10 : 90 to 90 : 10, more preferably in a range of 20 : 80 to 80 : 20, and most preferably in a range of 25 : 75 to 50 : 50. Within these ranges, it is easy to provide a lactase preparation having a plurality of properties.

When the lactase preparation of the present embodiment has a salt or an ion thereof, the lactase activity is improved. Examples of the salt include magnesium chloride, sodium chloride, potassium chloride, calcium chloride, and manganese chloride. Examples of the ion include monovalent cations such as sodium and potassium, and divalent cations such as magnesium, manganese, and calcium. In particular, when the lactase preparation of the present embodiment has two or more salts selected from a group consisting of magnesium chloride, sodium chloride, potassium chloride, calcium chloride, and manganese chloride, or ions thereof, the lactase activity is remarkably improved.

The use of the lactase preparation having a salt or an ion thereof is not limited as long as the lactase preparation promotes lactose decomposition, and examples of the use of the lactase preparation include adding or including the lactase preparation in an aqueous lactose solution as well as a raw material milk such as cow's milk. Even in a case where the lactase preparation does not contain the above-described salt or ion thereof, the lactose decomposition rate in a reaction system can be increased as long as the reaction system contains the above-described salt or ion thereof. For example, the lactose decomposition rate can be accelerated by further adding the above-described salt as an ion to a reaction system in which an active ingredient (lactase) is added to an aqueous lactose solution.

From a viewpoint of accelerating the lactose decomposition rate, a lower limit of the content of the salt contained in the lactase preparation is preferably 0.1 mM or more, more preferably 0.5 mM or more, and may be 1 mM or more, 10 mM or more, and still more preferably 40 mM or more.

An upper limit of the content of the salt is preferably 1000 M or less, more preferably 500 M or less, and may be 100 M or less, and still more preferably 50 M or less. As an upper limit value and a lower limit value of the content of the salt contained in the lactase preparation, the above ranges can be appropriately combined.

A lower limit of the monovalent cation contained in the lactase preparation is preferably 0.1 mM or more, more preferably 1 mM or more, and may be 10 mM or more, 100 mM or more, and still more preferably 500 mM or more. An upper limit of the monovalent cation is preferably 1000 M or less, more preferably 500 M or less, may be 100 M or less, and still more preferably 50 M or less. As an upper limit value and a lower limit value of the monovalent cation contained in the lactase preparation, the above ranges can be appropriately combined.

A lower limit of the divalent cation contained in the lactase preparation is preferably 0.1 mM or more, more preferably 1 mM or more, may be 10 mM or more, and still more preferably 40 mM or more. An upper limit of the divalent cation is preferably 100 M or less, more preferably 20 M or less, may be 10 M or less, and still more preferably 2 M or less. As an upper limit value and a lower limit value of the divalent cation contained in the lactase preparation, the above ranges can be appropriately combined.

From a viewpoint of obtaining a sufficient lactose decomposition rate by a salt, the pH of the lactose aqueous solution is typically 4.5 to 9.0.

A shape of the lactase preparation is not limited. It may be in a solid or liquid form at room temperature. In a case where the lactase preparation is used for a solid substance, the lactase preparation is preferably in a solid form, and in a case where the lactase preparation is used in a liquid (for example, cow's milk), the lactase preparation is preferably in a liquid form.

### (Protein amount)

A lower limit of the content of the protein contained in the lactase preparation of the present embodiment is preferably 0.072 mg/mL or more in the case of a liquid lactase preparation, and preferably 0.72 mg/g or more in the case of a solid lactase preparation. On the other hand, an upper limit value of the content of the protein is not particularly limited, but is preferably, for example, 720 mg/mL or less.

When the content of the protein is equal to or more than the lower limit value, a desired activity can be secured even if the storage time is prolonged.

The amount of protein contained in the lactase preparation of the present embodiment can be measured by Bradford method.

### (Activity value)

The lactase preparation of the present embodiment desirably has a lactase activity of 10 to 100,000 LYU/mL, more desirably has an activity of 100 to 90,000 LYU/mL, and still more desirably has an activity of 1,000 to 80,000 LYU/mL.

The method for measuring the activity is as follows. A lactase preparation (an enzyme liquid) to be measured is added to a lactose solution (final concentration: 10%) dissolved in a 0.1 M phosphate buffer solution (pH 6.5) containing a 0.1 mM manganese chloride solution, and the lactase preparation is held at pH 6.5 and 37°C for 10 minutes. The reaction is terminated by the addition of 1.5 N sodium hydroxide solution. The reaction solution is allowed to cool to room temperature and the solution is neutralized by adding 1.5 N hydrochloric acid. Glucose CII Test Wako (manufactured by Wako Pure Chemical Industries, Ltd.) is used for quantification of the amount of free glucose in the reaction solution. The amount of enzyme that produces 1 µmol of glucose per minute under these conditions is defined as 1 LYU.

### (Stabilizer)

In the lactase preparation of the present embodiment, the content of the stabilizer is preferably 10 to 90 mass%, more preferably 20 to 80 mass%, and still more preferably 30 to 70 mass%.

Examples of the stabilizer include sorbitol and glycerin.

### <Method for producing lactose-decomposed milk>

### (Raw material milk)

The milk in which the lactase or lactase preparation of the present embodiment is used is not particularly limited as long as it contains lactose. Examples of the origin of milk include cows, goats, and sheep. Among them, it is preferable to use milk with an origin of cows.

The milk may be used as it is, and water, whole milk powder, skim milk powder, cream powder, whey powder, protein concentrated whey powder, butter milk powder, sugar-added milk powder, prepared milk powder, and the like may be added as necessary. It is also possible to adjust the solid content, the amount of lactose, and the like described later. In the present embodiment, the milk obtained by adding a material other than an enzyme to the above milk is referred to as a raw material milk.

The solid content contained in the raw material milk may be 0.1 mass% or more and 30 mass% or less, 0.5 mass% or more and 20 mass% or less, 1 mass% or more and 10 mass% or less, 2 mass% or more and 8 mass% or less, preferably 4.0 mass% or more, more preferably 6.0 mass% or more, and still more preferably 8.0 mass% or more. An upper limit value of the solid content contained in the raw material milk is not particularly limited, and is, for example, 30 mass%.

The amount of lactose contained in the raw material milk may be 0.1 mass% or more and 30 mass% or less, 0.5 mass% or more and 20 mass% or less, 1 mass% or more and 10 mass% or less, or 2 mass% or more and 8 mass% or less, and is preferably 4.0 mass% or more and 5.8 mass% or less, preferably 4.4 mass% or more and 5.4 mass% or less, and more preferably 4.8 mass% or more and 5.0 mass% or less.

The amount of protein contained in the raw material milk may be 0.1 mass% or more and 30 mass% or less, 0.5 mass% or more and 20 mass% or less, 1 mass% or more and 10 mass% or less, or 2 mass% or more and 8 mass% or less, and is preferably 2.0 mass% or more and 4.8 mass% or less, more preferably 2.6 mass% or more and 4.2 mass% or less, and still more preferably 3.2 mass% or more and 3.6 mass% or less.

The amount of fat contained in the raw material milk may be 0.1 mass% or more and 30 mass% or less, 0.5 mass% or more and 20 mass% or less, 1 mass% or more and 10 mass% or less, or 2 mass% or more and 8 mass% or less, and is preferably 1.5 mass% or more, more preferably 2.5 mass% or more, and still more preferably 3.5 mass% or more.

The sterilization method of the raw material milk is not particularly limited, and examples thereof include low temperature long time pasteurization (LTLT), high temperature long time sterilization (HTLT), and ultra-high temperature sterilization (UHT) in an order of low heat history. Among these sterilization methods, UHT is preferably applied to sterilization of milk. The raw material milk may be non-sterilized raw milk.

The lactase or lactase preparation of the present embodiment is added to the raw material milk at a predetermined concentration, and then reacted at a predetermined temperature for a predetermined time, whereby a lactose-decomposed milk having a high lactose decomposition rate can be produced. It is preferable to use cow's milk as the raw material milk.

The reaction time is, for example, 1 hour to 48 hours, preferably 12 hours to 24 hours.

The reaction temperature is, for example, more than 0°C to 40°C, preferably 1°C to 25°C, and 1°C to 10°C.

The concentration of lactose contained in cow's milk is about 4.7 mass%. The amount of lactose contained in the lactose-decomposed milk is preferably 0.1 mass% or less (lactose decomposition rate: 97.87% or more), and more preferably 0.01 mass% or less (lactose decomposition rate: 99.79% or more). This makes it possible to sell lactose-free milk.

When the lactase in the raw material milk is 1.4 LYU/mL or more, a lactose-decomposed milk having a lactose amount of 0.1 mass% or less (lactose decomposition rate of 97.87% or more) is easily obtained, which is preferable.

When the lactase in the raw material milk is 2.1 LYU/mL or more, a lactose-decomposed milk having a lactose amount of 0.01 mass% or less (lactose decomposition rate of 99.79% or more) is easily obtained, which is preferable.

Although the embodiments of the present invention have been described above, these are examples of the present invention, and various configurations other than the above can be adopted.

### Examples

Hereinafter, the present invention will be described with reference to the examples and comparative examples, but the present invention is not limited thereto.

### <Enzyme used>

The lactases used for the evaluation are shown below.
BL105A_1453: [E. coli recombinant expression purified enzyme derived from Bifidobacterium longum 105-A strain]
YNL 2: [derived from Kluyveromyces lactis, manufactured by GODO SHUSEI CO., LTD., # 21006]
YNL 2LS: [derived from Kluyveromyces lactis, manufactured by GODO SHUSEI CO., LTD., # 21002]
Lactase A: [derived from Kluyveromyces lactis]
Lactase B: [derived from Bifidobacterium bifidum]
Lactase C: [derived from Lactobacillus delbrueckii ssp. bulgaricus]

### <Production of crude enzyme liquid of BL105A_1453>

E. coli BL21 (DE3) transformant into which expression plasmid pET 23a-BL105A 1453 of BL105A 1453 was introduced was inoculated into a test tube containing 5 mL of LB Amp medium (50 µg/mL) and cultured overnight at 18°C. 250 µL of a culture solution was inoculated into a 100 mL conical flask containing 25 mL of TB Amp medium (50 µg/mL), and cultured with shaking at 18°C. This was performed for 10 tubes. Using an absorptiometer, when the absorbance A₆₁₀ reached 0.5, isopropyl-β-thiogalactopyranoside (IPTG) was added so as to have a final concentration of 0.5 mM to induce the production of recombinant protein. Induction culture was performed at 18°C for 24 hours. The bacterial cells were recovered from the culture solution by centrifugation (8,000 rpm, 10 min, 4°C), suspended in 10 mL of a binding buffer (20 mM sodium phosphate buffer solution, pH 7.5, 500 mM sodium chloride) of Ni column chromatography, and the bacterial cells were disrupted by sonication (URTRA SONIC DISRUPTOR UR-200P, manufactured by Tomy Seiko Co., Ltd.). The supernatant obtained by centrifugation (8,000 rpm, 10 min, 4°C) was used as a crude enzyme liquid containing BL105A_1453.

### <Production of purified enzyme liquid of BL105A_1453>

A Ni-NTA Agarose column (Φ1.0 cm× 4.0 cm; QIAGEN) in which the crude enzyme liquid obtained above was equilibrated with binding buffer is provided. Non-adsorbed proteins were removed with a washing buffer (20 mM sodium phosphate buffer solution, pH 7.5, 500 mM sodium chloride, 50 mM imidazole), and then adsorbed proteins were eluted with a 20-500 mM imidazole linear concentration gradient (an elution buffer A, 20 mM sodium phosphate buffer solution, pH 7.5, 20 mM imidazole; an elution buffer B, 20 mM sodium phosphate buffer solution, pH 7.5, 500 mM imidazole; 60 mL each). The amount of a liquid fraction was 8 mL for washing and 3 mL for elution. Fractions (elution fra. 7-14) whose activity was confirmed by the ONPG degradation activity plate assay were collected. The collected fractions were dialyzed against 1 L of 10 mM sodium phosphate buffer solution (pH 6.5) using a dialysis membrane (cellulose tube 36/32; Viskase Companies, Inc.) once, and further dialyzed against 5 L of the same buffer solution twice. After dialysis, it was concentrated to 1.5 mL using Amicon Ultra-15 (MWCO, 100,000; Merck Millipore). This was used as a BL105A_1453 purified enzyme liquid. Glycerol was added to the obtained purified enzyme liquid so as to have a final concentration of 50%, and the mixture was stored at -80°C. The purified enzyme liquid was subjected to SDS-PAGE to confirm a single band.

### <Temporal change of lactose decomposition in cow's milk>

A lactose decomposition test was performed on the BL105A_1453 purified enzyme liquid and other lactase preparations (enzyme liquids) prepared above. 1 mL of commercially available cow's milk (manufactured by Meiji Co., Ltd., lactose content: 4.7 mass%) was dispensed into a 1.5 mL Eppendorf tube and pre-incubated at 10°C for 10 minutes. To this was added 20 µL of each enzyme liquid prepared using a buffer solution with each lactase so as to have a final concentration of 0.01, 0.02, 0.03, 0.04, 0.06, 0.08, 0.12 and 0.16 v/v%, and the mixture was kept at 10°C for 48 hours. As a blank, one obtained by adding 20 µL of a buffer solution to 1 mL of cow's milk was used. For BL105A_1453, a product (a make-up) activity was assumed to be 3500 LYU/mL. The reaction was stopped by adding 20% sulfosalicylic acid in an amount of 1/20. As a buffer solution, a 0.1 M phosphate buffer solution (pH 6.5) containing 0.1 mM manganese chloride was used.

### <Method for calculating lactose decomposition rate>

Sugar analysis of each sample using HPLC and HPAEC-PAD was performed according to the following.

### «HPLC analysis»

A sample diluted 5 folds with ultrapure water was filtered through a filter having a pore size of 0.22 µm, and a filtrate was analyzed using HPLC. Conditions during HPLC analysis are as follows.
Main body: high-performance liquid chromatography (HPLC) Waters 2696
Column: CARBOSep CHO-620CA (ϕ6.5 mm × 300 mm) manufactured by Transgenomic, Inc., 85°C
Mobile phase: water
Flow rate: 0.4 mL/min
Detector: RI
Sample injection amount: 5 µL

### «HPAEC-PAD analysis»

A sample diluted 250 folds with ultrapure water was filtered through a 0.22 µm filter and the filtrate was analyzed using HPAEC-PAD. Conditions during HPAEC-PAD analysis are as follows.
Main body: ICS-6000⁺, AS-Ap autosampler manufactured by Dionex Corporation
Column: CarboPac PA1 (ϕ4.0 mm × 250 mm) manufactured by Dionex Corporation, 20°C
Mobile phase: gradient of the following four liquids
A: 100 mM sodium hydroxide solution
B: 600 mM sodium acetate solution containing 100 mM sodium hydroxide
C: Water
D: 50 mM sodium acetate solution
Flow rate: 1.0 mL/min
Detector: pulsed amperometry, 30°C
Sample injection amount: 25 µL

For each sample in which decomposition of lactose was confirmed to be about 50% or more by HPLC analysis, an analysis value obtained by HPAEC-PAD analysis was used for calculation of the lactose decomposition rate. The lactose decomposition rate was calculated by comparing the residual lactose concentration of each sample with the lactose concentration of cow's milk analyzed under the same conditions.

### <Lactase activity (LYU/mL) at which lactose concentration in cow's milk reaches 0.1 mass% or 0.01 mass%>

The lactase activity (LYU/mL) at which the lactose concentration in cow's milk reached 0.1 mass% or 0.01 mass% at 10°C for each reaction time is shown in Table 1. This lactase activity indicates the final concentration in cow's milk.

**[Table 1]**

| Enzyme | Lactase activity required to make lactose concentration reach 0.1% or less (LYU/mL) | | | Lactase activity required to make lactose concentration reach 0.01% or less (LYU/mL) | | |
|---|---|---|---|---|---|---|
| | 16 hr | 24 hr | 48 hr | 16 hr | 24 hr | 48 hr |
| BL105A_1453 | 2.10 | 1.40 | 0.70 | 4.20 | 2.10 | 1.05 |
| YNL 2 | 7.12 | 4.74 | 2.37 | 18.9 | 14.2 | 4.74 |
| YNL 2LS | 7.66 | 3.83 | 2.55 | 20.4 | 10.2 | 5.11 |
| Lactase A | 4.59 | 3.44 | 2.29 | 9.18 | 6.88 | 3.44 |
| Lactase B | 2.21 | 1.66 | 0.83 | 3.31 | 2.21 | 1.10 |
| Lactase C | 3.72 | 2.79 | 1.86 | 14.8 | 14.8 | 3.72 |

### <Lactose resolution per amount of protein included in measurement target at 10°C>

In order to compare the lactose resolution per amount of protein contained in the measurement target, a specific activity was calculated using the LYU activity as an index. Protein quantification of BL105A_1453 enzyme liquid and other lactase preparations followed the Bradford method.

The obtained specific activity values and the results in Table 1 were combined to calculate the amounts of protein required to adjust the lactose concentration to 0.1% and 0.01%. BL105A_1453 was confirmed to have the highest superiority in the 16 to 48-hour reaction among the lactases compared this time (see Table 2).

**[Table 2]**

| Enzyme | Specific activity (LYU/mg) | Protein required to make lactose concentration reach 0.1% or less (mg/L) | | | Protein required to make lactose concentration reach 0.01% or less (mg/L) | | |
|---|---|---|---|---|---|---|---|
| | | 16 hr | 24 hr | 48 hr | 16 hr | 24 hr | 48 hr |
| BL105A_1453 | 139 | 15.1 | 10.1 | 5.0 | 30.2 | 15.1 | 7.6 |
| YNL 2 | 215 | 33.1 | 22.1 | 11.0 | 88.2 | 66.2 | 22.1 |
| YNL 2LS | 254 | 30.2 | 15.1 | 10.1 | 80.4 | 40.2 | 20.1 |
| Lactase A | 291 | 15.8 | 11.8 | 7.9 | 31.5 | 23.7 | 11.8 |
| Lactase B | 43 | 51.0 | 38.2 | 19.1 | 76.5 | 51.0 | 25.5 |
| Lactase C | 130 | 28.5 | 21.4 | 14.3 | 114 | 114 | 28.5 |

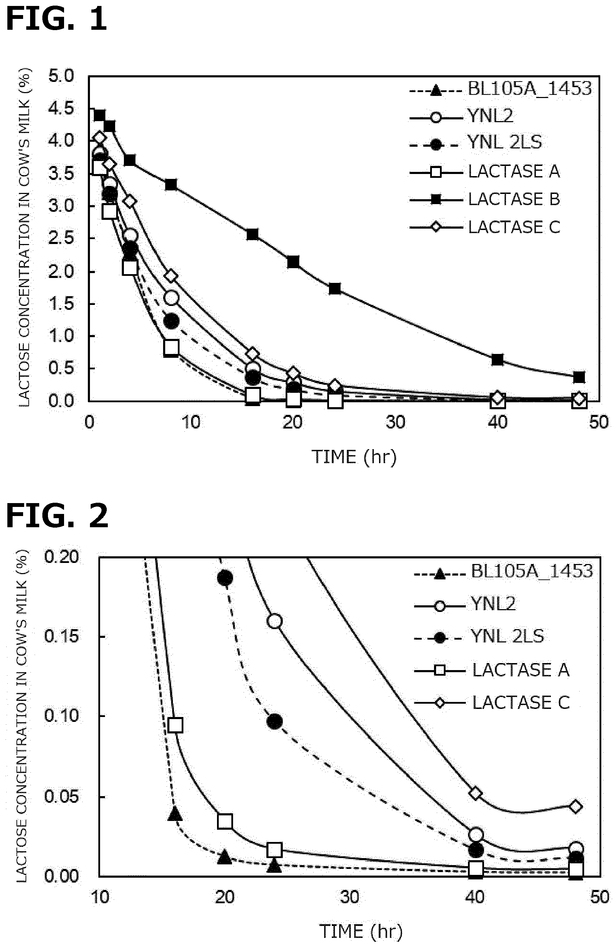

Fig. 1 and Fig. 2 (a partially enlarged view of Fig. 1) are graphs showing changes in the residual lactose concentrations at 10°C when BL105A_1453 and other lactases are added to 1 mL of cow's milk in an amount such that the amount of protein is about 15.1 µg. As shown in Figs. 1 and 2, in the case of BL105A_1453 purified enzyme liquid, the residual lactose concentrations in cow's milk at 10°C after 16 and 24 hours were 0.1% or less and 0.01% or less, respectively, and thus the lactose decomposition rate was higher than those of the other prepared lactase preparations.

### «Analysis of activators in cow's milk»

The activators in cow's milk were analyzed for the YNL 2 purified enzyme liquid and the BL105A_1453 purified enzyme liquid.

Specifically, 10 µL of the enzyme liquid was added to 190 µL of the solution obtained under each of the following conditions, and then the lactose decomposition rate (the reaction rate) was calculated in the reaction system that was held at 10°C or 37°C for 10 minutes.
Condition 1: 139 mM lactose, 0.5 mM MES-NaOH (pH = 6.5)
Condition 2: 139 mM lactose (Na⁺: 18.4 mM, K⁺: 39.6 mM, Mg²⁺: 4.25 mM) containing mixed salt (NaCl, KCl, and MgCl₂)
Condition 3: cow's milk
Condition 4: fractionated cow's milk
Condition 5: desalted fractionated cow's milk - 1 (charged components having a molecular weight < 100 of condition 4 are removed by electrodialysis)
Condition 6: desalted fractionated cow's milk - 2 (charged components of 4 are removed by an ion exchange resin)

The lactose decomposition rate was calculated by the following procedure.

After 10 µL of the enzyme liquid was added to 190 µL of the solution obtained under each of the above conditions, the mixture was reacted at 10°C or 37°C for 10 minutes, and then 10 µL of 21% sulfosalicylic acid was added thereto to stop the reaction. To 50 µL of a solution obtained by diluting a sample supernatant collected by centrifugation 10 folds with ultrapure water, 100 µL (pH 7.0) of a 2 M tris-hydrochloric acid buffer solution and 20 µL of a glucose quantification reagent (Glucose CII Test Wako) were added, and the mixture was kept at 37°C for 1 hour to measure A₅₀₅. Glucose concentration was calculated from A₅₀₅ based on calibration curves (0-600 µM glucose and A₅₀₅). For the calibration curves, a mixed solution of glucose aqueous solution : cow's milk supernatant = 9 : 1 was used. The amount of enzyme hydrolyzing 1 µmol of lactose per minute under these conditions was defined as 1 unit. The obtained results are shown in Tables 3 and 4.

**[Table 3]**

| **10°C** | Unit: *µ* mol/min/mg | |
|---|---|---|
| | **YNL 2** | **BL105A_1453** |
| **Condition 1** | 3.31 | 6.01 |
| **Condition 2** | 35.0 | 27.6 |
| **Condition 3** | 19.8 | 38.0 |
| **Condition 4** | 17.6 | 24.2 |
| **Condition 5** | 0.202 | 5.95 |
| **Condition 6** | 2.73 | 15.2 |

**[Table 4]**

| **37°C** | Unit: *µ* mol/min/mg | |
|---|---|---|
| | **YNL 2** | **BL105A_1453** |
| **Condition 1** | 0.469 | 17.7 |
| **Condition 2** | 250 | 254 |
| **Condition 3** | 152 | 312 |
| **Condition 4** | 142 | 253 |
| **Condition 5** | 2.54 | 21.8 |
| **Condition 6** | 3.54 | 74.6 |

It was determined that the salt in the cow's milk greatly contributed to the activation of the enzyme since the activity of YNL 2 was reduced to a level of almost 139 mM lactose by the electrodialysis of the fractionated cow's milk, and the activity exceeded the level in the cow's milk by an addition of the mixed salt.

For BL105A_1453, the activity decreased to the level of 139 mM lactose when the fractionated cow's milk was desalted with the ion exchange resin, but the decrease in activity was mild when the fractionated cow's milk was desalted by electrodialysis. Since the addition of the mixed salt results in a level of activity in cow's milk, it is considered that the salt in cow's milk acts on activation, but it is also considered that a component that is not removed by electrodialysis may act as a substitute for the salt.

### «Evaluation of effect of metal salt on BL105A_1453»

The o-nitrophenyl-β-D-galactopyranoside (ONPG) decomposition rate (the reaction rate) at 37°C in each of the following reaction systems was calculated.
Reaction system 1: (Na⁺) (50 µL)
2 mM ONPG, 10 mM MES-NaOH (pH 6.0, containing 5.3 mM Na⁺), 0-50 mM NaCl, and enzyme
Reaction system 2: (K⁺, Mg²⁺, Mn²⁺, Ca²⁺) (50 µL)
2 mM ONPG, 10 mM MES-NaOH (pH 6.0, containing 6 mM K⁺), 0-50 mM KCl, 0-10 mM MgCl₂, 0-0.1 mM MnCl₂ or 0-50 mM CaCl₂, and enzyme

The ONPG decomposition rate was calculated by the following procedure.

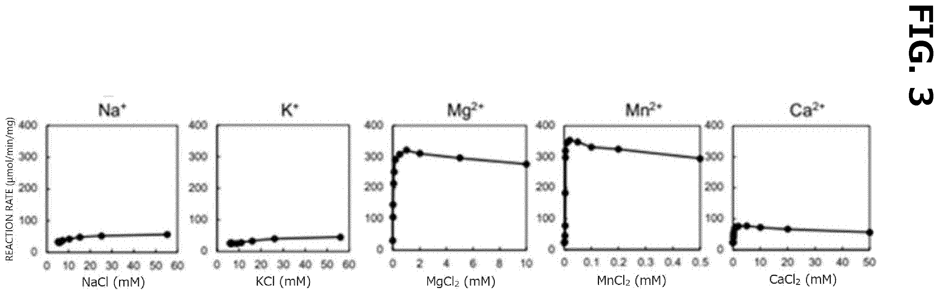

The reaction system 1 or 2 was reacted at 37°C for 10 minutes, 100 µL of a 1 M sodium carbonate solution was then added, and A₄₀₀ was measured. The amount of enzyme that degrades 1 µmol of ONPG per minute under these conditions was defined as one unit. The obtained results are shown in Fig. 3.

### <Enzymological properties of BL105A_1453>

### <<Optimum pH>>

The optimum pH of lactase was measured by the following method.

150 µL of the BL105A_1453 enzyme liquid diluted with a buffer solution (0.1 M sodium phosphate buffer solution containing 0.1 mM manganese chloride, pH 4.5 to 9.5) adjusted to each pH was taken in a 1.5 mL tube, mixed with 150 µL of the buffer solution, and then pre-incubated at 37°C for 3 minutes. The mixture was reacted with 300 µL of a substrate (ONPG 100 mg/distilled water 100 mL) at 37°C for 1 minute, and 600 µL of 0.2 M sodium carbonate was added to stop the reaction. A value of lactase activity was calculated from an absorbance of the reaction solution at OD₄₂₀. One unit of lactase activity was defined as the amount of enzyme required to change a measured value by one every 10 minutes.

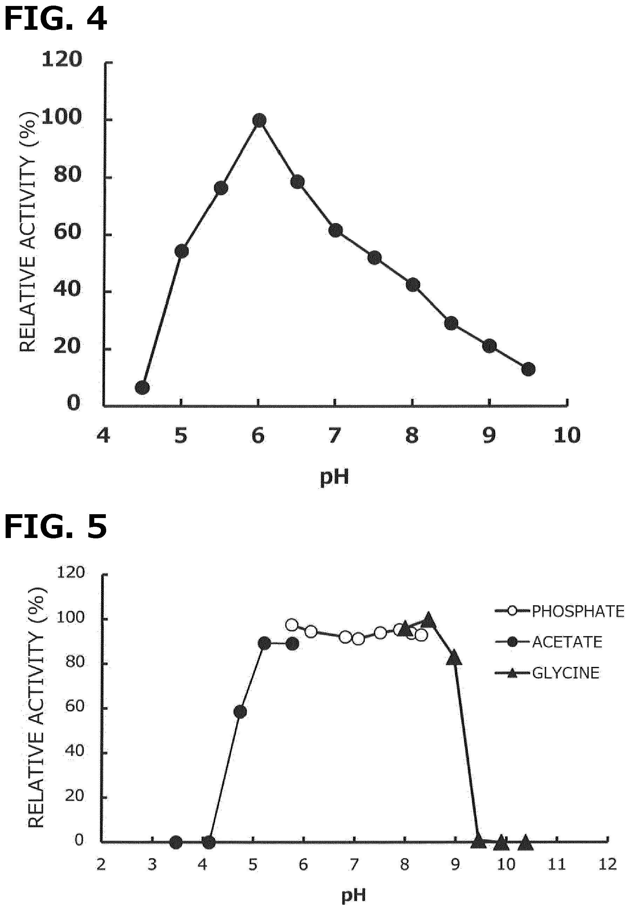

The obtained results are shown in Fig. 4. The optimum pH of BL105A_1453 was 6.0.

### «pH stability»

The pH stability of lactase was measured by the following method.

The BL105A_1453 enzyme liquid was diluted 10 folds with each buffer solution (pH 3.0 to 10.5) and incubated at 37°C for 30 minutes. After cooling on ice for 3 minutes, it was further diluted 200 folds with a buffer solution at pH 6.0. 300 µL of the diluted enzyme liquid was taken in a 1.5 mL tube, mixed with 300 µL of a buffer solution, and then pre-incubated at 37°C for 3 minutes. The mixture was reacted with 600 µL of a substrate (ONPG 100 mg/distilled water 100 mL) at 37°C for 1 minute, and 1200 µL of 0.2 M sodium carbonate was added to stop the reaction. A value of lactase activity was calculated from an absorbance of the reaction solution at OD₄₂₀. One unit of lactase activity was defined as the amount of enzyme required to change a measured value by one every 10 minutes.

The pH stability of lactase can be measured by the same method as the method shown for the optimum pH of lactase except for the buffer solutions to be used and incubation at 37°C for 30 minutes.

The buffer solution at each pH was as follows, and the pH was plotted as the measured value during enzyme dilution.
- pH 3.5 to 5.5
   : 0.1 M sodium acetate buffer solution containing 0.1 mM manganese chloride
- pH 5.5 to 9.0
   : 0.1 M sodium phosphate buffer solution containing 0.1 mM manganese chloride
- pH 8 to 10.5
   : 0.1 M glycine-sodium hydroxide buffer solution containing 0.1 mM manganese chloride

The obtained results are shown in Fig. 5. A stability range of BL105A_1453 was pH 5.2 to 8.4.

### <<Optimum temperature>>

The optimum temperature of lactase was measured by the following method.

300 µL of a BL105A_1453 enzyme liquid diluted 2000 folds with a sodium phosphate buffer solution having a pH of 6.0 was taken, mixed with 300 µL of a buffer solution, and then pre-incubated at 4, 10, 15, 20, 25, 30, 35, 37, 40, 45, 50, 55, and 60°C for 3 minutes. Thereafter, the activity was measured at each temperature in the same manner as in a pH stability test.

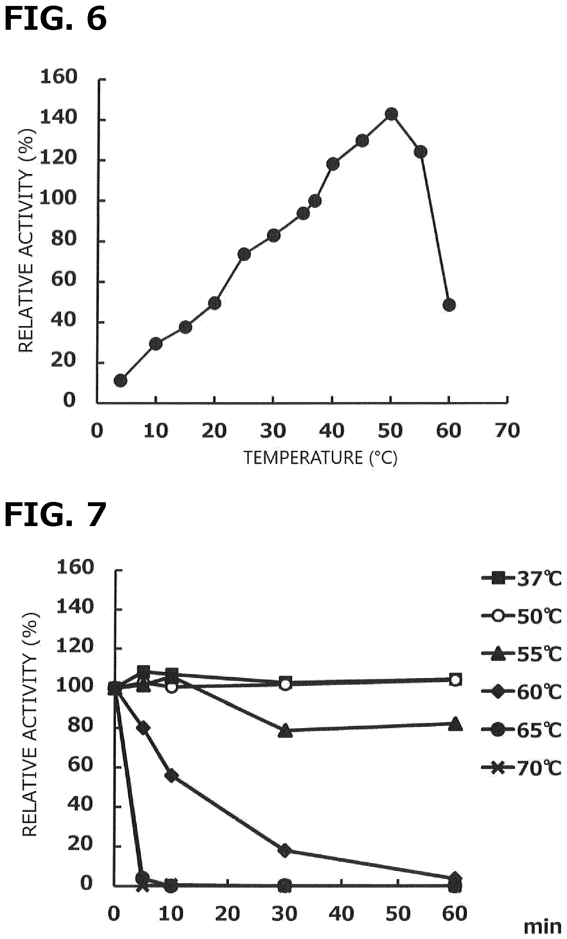

The obtained results are shown in Fig. 6. In Fig. 6, the activity at 37°C was used as a reference (relative activity: 100%). The optimum temperature of BL105A_1453 was 50°C.

### <<Temperature stability>>

The temperature stability of lactase was measured by the following method.

The BL105A_1453 enzyme liquid was diluted 10 folds with a buffer solution at pH 6.0 and incubated at 37, 50, 55, 60, 65, and 70°C for 0, 5, 10, 30, and 60 minutes. After cooling on ice for 3 minutes, it was further diluted 200 folds with a buffer solution at pH 6.0. 300 µL of the diluted enzyme liquid was taken in a test tube, mixed with 300 µL of a buffer solution, and then pre-incubated at 37°C for 3 minutes. Thereafter, the activity was measured in the same manner as in the pH stability test.

The obtained results are shown in Fig. 7. BL105A_1453 showed 100% of lactase residual activity even after reaction at 50°C for 1 hour.

### <Production of galacto-oligosaccharides>

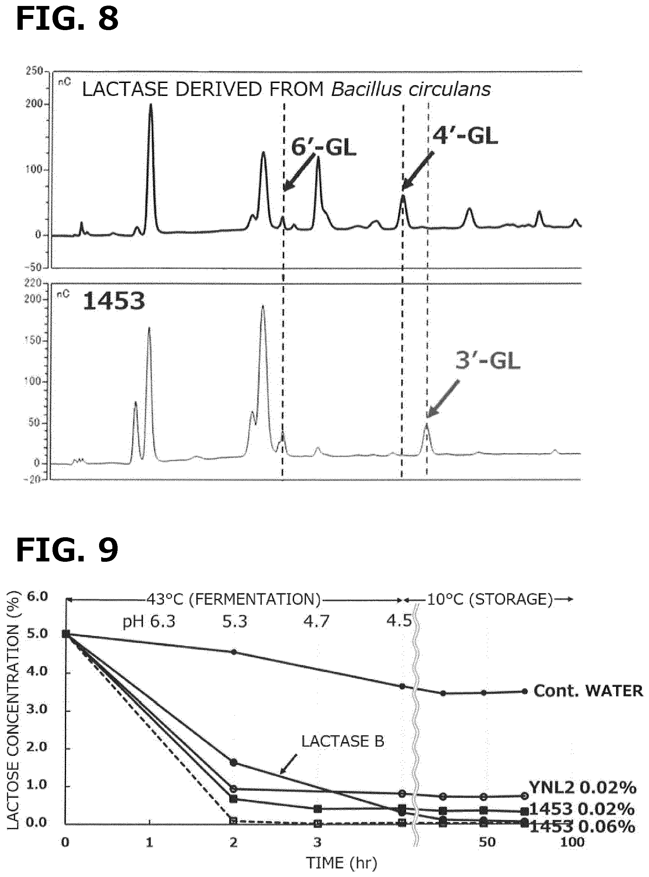

When BL105A_1453 was reacted in the presence of 30 or 50 w/v% lactose, it was confirmed that galacto-oligosaccharides of 3 or more saccharides were produced at a maximum of 20% (as a yield of galacto-oligosaccharides). A major structure of galacto-oligosaccharides produced by BL105A_1453 was 3'-galactosyllactose (in a lower part of Fig. 8). As shown in an upper part of Fig. 8, since the main structure of the galacto-oligosaccharide-producing enzyme derived from Bacillus circulans (product name: Biolacta) was 4'-galactosyllactose, it was confirmed that the types of galacto-oligosaccharides to be produced were different.

Similarly to 4'-galactosyllactose, 3'-galactosyllactose is a substance expected to have a bifidobacterium growth effect in a human intestine.

Here, when a trisaccharide galacto-oligosaccharide is produced, one molecule of glucose and one molecule of trisaccharide galacto-oligosaccharide are produced from two molecules of lactose. A theoretical yield of galacto-oligosaccharides at this time is about 74 mass%. In a case where a tetrasaccharide galacto-oligosaccharide is produced, two molecules of glucose and one molecule of tetrasaccharide galacto-oligosaccharide are generated from three molecules of lactose. A theoretical yield of galacto-oligosaccharides at this time is about 65 mass%. Therefore, when galacto-oligosaccharides are produced from lactose, it is most efficient in terms of yield to produce only trisaccharide galacto-oligosaccharides. Here, the yield (mass%) of galacto-oligosaccharides means a percentage obtained by dividing the "production amount of galacto-oligosaccharides" by the "consumption amount of lactose". The galacto-oligosaccharides produced can be measured by HPLC. Specifically, a method described in WO 2019/194062 may be adopted.

### <Production of fermented milk>

A starter (lactic acid bacterium YF-L812 strain 10 mg/100 g, bifidobacterium BB-12 strain 5 mg/100 g) was put in commercially available cow's milk (manufactured by Meiji Co., Ltd.), and the milk was stirred and dispensed, and then the BL105A_1453 enzyme liquid was added so as to have a product activity of 5200 LYU/mL of 0.02 and 0.06%, respectively. As controls, a group to which YNL 2 0.02% was added and a group to which lactase B (enzyme derived from Bifidobacterium bifidum) 0.02% was added were provided. Sampling was performed at 0, 2, and 4 hours after the start of fermentation, and the residual lactose concentration (mass%) was calculated by sugar analysis. However, in a group to which BL105A_1453 was added, the fermentation proceeded quickly, and therefore sampling was also performed after 3 hours had elapsed. After completion of fermentation, the samples were transferred to 10°C, and the residual lactose concentrations after 24, 48, and 72 hours were calculated. All samples to be subjected to sugar analysis were diluted 3 folds with water, and deproteinized with sulfosalicylic acid. Other conditions for sugar analysis followed those described above in «HPLC analysis».

Fig. 9 shows the changes in the lactose concentrations of the fermented milk obtained as described above. In a case where a comparison was made with the same addition amount (0.02% addition group), the decomposition rate of BL105A_1453 was equivalent to that of lactase B at the end of fermentation (after 4 hours), and the lactose concentration in the sample was 0.4%. On the other hand, in a case where 0.06% of BL105A 1453 was added, the lactose concentration reached 0.1% or less after two hours of fermentation. Only lactase B reacted even under low pH conditions at 10°C after the end of fermentation, and the lactose concentration reached 0.1% or less 48 hours after the start of fermentation. Since the activity of BL105A_1453 decreased at a pH of 4.7 or lower in the pH stability test described above, it was considered that BL105A_1453 started to be deactivated at the end of fermentation.

From the above results, it is possible to efficiently decompose lactose in a fermentation stage by the method of adding BL105A_1453 simultaneously with the starter to obtain a fermented milk. This makes it possible to stably produce a lactose-free fermented milk without requiring a separate step in a fermented milk production step.

### <Lactose decomposition test in cow's milk (10°C) combining YNL 2 and BL105A_1453>

The BL105A_1453 and YNL 2 preparations were combined to perform a lactose decomposition test in cow's milk. 20 µL of each enzyme was added to cow's milk so as to have a final concentration of 0.04% (0.04% of YNL 2 and 1453 combined), and the mixture was held at 10°C for 48 hours. At this time, the product activity of BL105A_1453 was assumed to be 5,200 LYU/mL. Other conditions and sugar analysis procedure followed those described above in <Temporal change of lactose decomposition in cow's milk>, <Method for calculating lactose decomposition rate>,

### «HPLC analysis», and «HPAEC-PAD analysis».

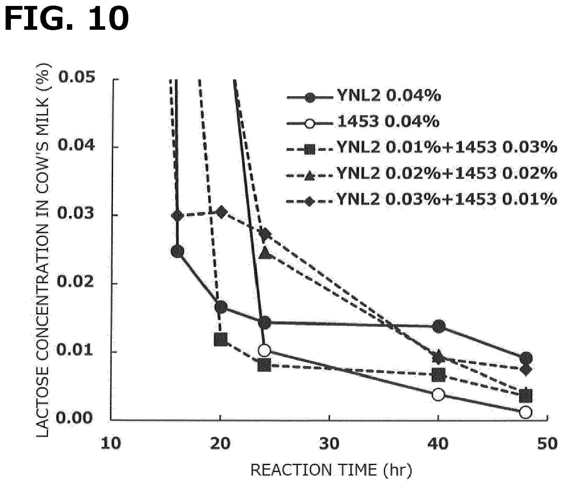

The obtained results are shown in Fig. 10. It shows a faster initial rate in the lactose decomposition in a YNL 2 concentration-dependent manner, and a higher final decomposition rate in a BL105A_1453 concentration-dependent manner. In Fig. 10, a lactose concentration of less than 0.01% is a lactose-free region.

From the above results, it was confirmed that a desired result is easily obtained by using a plurality of enzymes as the lactase.

## Claims

1. A lactase having, as lactase activity, any one of activities (1) to (6) or a combination of two or more of activities (1) to (6) below:
(1) when the lactase is added to cow's milk, an added amount of protein in the lactase at which a residual lactose concentration reaches 0.1% or less after 16 hours at 10°C is 15.1 mg/L or less;
(2) when the lactase is added to cow's milk, the added amount of protein in the lactase at which the residual lactose concentration reaches 0.01% or less after 16 hours at 10°C is 30.2 mg/L or less;
(3) when the lactase is added to cow's milk, the added amount of protein in the lactase at which the residual lactose concentration reaches 0.1% or less after 24 hours at 10°C is 10.1 mg/L or less;
(4) when the lactase is added to cow's milk, the added amount of protein in the lactase at which the residual lactose concentration reaches 0.01% or less after 24 hours at 10°C is 15.1 mg/L or less;
(5) when the lactase is added to cow's milk in an amount that makes a final concentration 1.4 LYU/ml, the residual lactose concentration reaches 0.1% or less after 24 hours at 10°C; and
(6) when the lactase is added to cow's milk in the amount that makes a final concentration 2.1 LYU/ml, the residual lactose concentration reaches 0.01% or less after 24 hours at 10°C.

2. The lactase according to claim 1, wherein the lactase is selected from any one of (i) to (iii) below:
(i) a protein consisting of an amino acid sequence represented by SEQ ID NO:1;
(ii) a protein consisting of an amino acid sequence in which one to several amino acid residues of the amino acid sequence represented by SEQ ID NO:1 are deleted, substituted, or inserted, and having lactase activity; and
(iii) a protein consisting of an amino acid sequence having 70% or more of identity to the amino acid sequence represented by SEQ ID NO:1 and having lactase activity.

3. The lactase according to claim 1 or 2, wherein the cow's milk according to claim 1 satisfies one or more of requirements below:
(A) a solid content contained in cow's milk is 0.1 mass% or more and 30 mass% or less;
(B) an amount of lactose contained in cow's milk is 0.1 mass% or more and 30 mass% or less;
(C) an amount of protein contained in cow's milk is 0.1 mass% or more and 30 mass% or less; and
(D) an amount of fat contained in cow's milk is 0.1 mass% or more and 30 mass% or less.

4. A lactase preparation comprising:
the lactase according to any one of claims 1 to 3; and
at least one selected from water, a salt, an excipient, a suspension agent, a buffer agent, a stabilizer, a preservative, or a physiological saline.

5. The lactase preparation according to claim 4, wherein the salt is one or more selected from a group consisting of magnesium chloride, sodium chloride, potassium chloride and calcium chloride.

6. A lactase preparation comprising:
the lactase according to any one of claims 1 to 3, and a second lactase having a different origin or different properties from the lactase.

7. A gene encoding proteins (i), (ii), or (iii) below:
(i) a protein consisting of an amino acid sequence represented by SEQ ID NO: 1;
(ii) a protein consisting of an amino acid sequence in which one to several amino acid residues of the amino acid sequence represented by SEQ ID NO: 1 are deleted, substituted, or inserted, and having lactase activity; and
(iii) a protein consisting of an amino acid sequence having 70% or more of identity to the amino acid sequence represented by SEQ ID NO: 1 and having lactase activity.

8. The gene according to claim 7, the gene consisting of any one of DNAs (a) to (d) below:
(a) a DNA consisting of a base sequence set forth in SEQ ID NO:2;
(b) a DNA consisting of a base sequence in which one to several bases are deleted, substituted, or added in the base sequence set forth in SEQ ID NO:2 and encoding the protein having lactase activity;
(c) a DNA consisting of a base sequence having 70% or more of sequence identity to the base sequence set forth in SEQ ID NO:2 and encoding the protein having lactase activity; and
(d) a DNA that hybridizes with a DNA consisting of a base sequence complementary to the base sequence set forth in SEQ ID NO:2 under stringent conditions and encodes the protein having lactase activity.

9. A recombinant vector comprising the gene according to claim 7 or 8.

10. A transformant transformed with the recombinant vector according to claim 9.

11. A method for producing a lactose-decomposed milk, comprising:
a step of adding the lactase according to any one of claims 1 to 3 or the lactase preparation according to claim 4 or 5 to a raw material milk; and
a step of reacting the lactase or a lactase contained in the lactase preparation with the raw material milk for a predetermined time.
